# EUROPEAN PATENT APPLICATION

(11) **EP 4 589 600 A1**
(43) Date of publication of application: **23.07.2025**
(21) Application number: 25152680.2
(22) Date of filing: 17.01.2025
(51) Int. Cl.: G16H 30/40, G16H 50/20, G16H 50/50

(54) **GENERATING RESTORATION OPTIONS**

(30) Priority: 18.01.2024 US 202418415758
(71) Applicant: DENTSPLY SIRONA Inc., York, PA 17401 (US)
(72) Inventor: SCHNEIDER, Hans-Christian, 64625 Bensheim (DE)
(74) Representative: Taor, Simon Edward William

(57) **Abstract**

An aspect includes examining a dental defect in an oral cavity, performing a minimally invasive procedure to detect or remove a portion of the dental defect, scanning the oral cavity including a residual tooth substance remaining after the performing, to obtain an intraoral scan, computing one or more parameters of the dental defect or residual tooth substance, and generating a number of treatment plans for one or more restoration materials based on a triangulation of the dental defect and an enforcement of constraints of the one or more restoration materials. Each treatment plan includes a geometry of a restoration option, and an optimal restoration option is selected from the plurality of treatment plans.

## Description

### BACKGROUND

### Technical Field

The present disclosure generally relates to generating treatment plans and more particularly to generating restorations based on analyzing tooth defects, calculating possible treatment plans or restoration options based on geometry of the tooth defects and enforcement of material constraints, and selecting an optimal treatment plan or restoration option from the possible treatment plans or restoration options.

### Description of the Related Art

Traditional dental treatment planning may rely on assessment by dental professionals, often involving visual inspection, and subjective judgment. However, these methods may lack objectivity, efficiency, and precision as some dental practitioners and patients may not have all the available data to compare and contrast patient specific restorations options.

### BRIEF SUMMARY

According to an aspect, there is provided a system comprising: a processor; and a memory storing instructions that, when executed by the processor, configure the processor to: obtain intraoral scan data from an intraoral scan, the intraoral scan being of an oral cavity of a patient that includes a dental defect; compute one or more constraints for one or more restoration materials for the dental defect; use the intraoral scan data to generate a plurality of treatment plans for the one or more restoration materials based on geometry of the dental defect and enforcement of the one or more constraints of the one or more restoration materials, each treatment plan including a geometry of a restoration option; and select from the plurality of treatment plans an optimal restoration option.

In some embodiments, the dental defect comprises a tooth defect or a missing part of a tooth.

In some embodiments, the geometry (or morphology) of the dental defect is determined by triangulation of the dental defect. In some embodiments, the geometry (or morphology) of the dental defect is determined by surface tessellation.

In some embodiments, the geometry of the dental defect is determined by the processor using the intraoral scan data.

In some embodiments, the geometry of the dental defect forms part of the intraoral scan data.

In some embodiments, the determining the morphology of the dental defect comprises determining a volume occupied by the dental defect.

In some embodiments, the plurality of treatment plans are selected from the list consisting of a filling, an inlay, an onlay, and a crown.

In some embodiments, the generating the plurality of treatment plans comprises determining a vector representation of the dental defect, and comparing the vector representation of the dental defect to prestored reference vector representations of dental defects.

In some embodiments, the comparing the vector representation of the dental defect to prestored reference vector representations of dental defects comprises: selecting a group of prestored reference vector representations of dental defects and comparing the vector representation of the dental defect to this group prestored reference vector representations of dental defects, wherein the group of prestored reference vector representations is selected based on data related to the patient.

In some embodiments, the processor is configured to: propose, using a trained machine learning model, said optimal restoration option using characteristics of the plurality of treatment plans as inputs to the trained machine learning model.

In some embodiments, the characteristics of the plurality of treatment plans are selected from the list consisting of a tooth number, a caries susceptibility, a bruxism grade, a patient or dentist preference, a sacrifice to defect ratio parameter for each treatment plan that represents a volume of tooth material to remove for the treatment plan relative to a volume of the dental defect, and an estimated fracture force for each treatment plan;

In some embodiments, the optimal restoration option includes a durability of a treatment plan of the plurality of treatment plans.

In some embodiments, the one or more constraints is selected from a list consisting of a minimum wall thickness of the one or more restoration materials, a volume of a residual tooth substance to remove to achieve a treatment plan, an expected masticatory pressure based on tooth position or defect location, and a medical history of a patient.

In some embodiments, the processor is configured to: compute a volume of the residual tooth substance to remove to achieve a treatment plan and computing an invasiveness score based on at least the volume.

In some embodiments, the system comprises a server arranged to receive the intraoral scan data or receive an intraoral scan from a communicatively coupled scanning apparatus (e.g. a dental acquisition unit equipped with a scanner). In such cases, the server may then provide the optimal restoration option for display at a suitable display apparatus.

In some embodiments, the system further comprises a scanner arranged to perform the intraoral scan of the oral cavity. The intraoral scan may comprise obtaining one or more images or other scan data.

In some embodiments, the processor is configured to: for each treatment plan of the plurality of treatment plans display on a screen, a triangulation of a prepared tooth and/or a restoration of the treatment plan; display a sacrifice to defect ratio parameter representing a volume of material to remove relative to a volume of the dental defect; and display an expected fracture force of the restoration. The displaying may take place on a screen that is communicatively coupled to the system.

In some embodiments, the processor is configured to use the intraoral scan data to compute a proximity of residual tooth substance to a pulp or tooth root and computing an invasiveness score based on at least the proximity.

In some embodiments, the processor is configured to, for each treatment plan of the plurality of treatment plans, compute a restoration prognosis comprising a stability score and/or a predicted lifetime.

In some embodiments, the generating is further based on a preference selected from the list consisting of a material availability, a cost limit, a maximum invasiveness, a minimum restoration lifetime.

In some embodiments, the plurality of treatment plans are ordered according to a predefined metric and visualized.

In some embodiments, one or more other characteristics of the dental defect are also visualized.

In some embodiments, the generating is performed by one or more of machine learning, analytical/numerical computation, statistical analysis, and preference filtering.

According to an aspect, there is provided a non-transitory computer-readable storage medium, including instructions that when executed by a computer, cause the computer to: obtain intraoral scan data from an intraoral scan, the intraoral scan being of an oral cavity of a patient that includes a dental defect; compute one or more constraints for one or more restoration materials for the dental defect; use the intraoral scan data to generate a plurality of treatment plans for the one or more restoration materials based on morphology of the dental defect and enforcement of the one or more constraints of the one or more restoration materials, each treatment plan including a geometry of a restoration option; and select from the plurality of treatment plans an optimal restoration option.

In one aspect, a method includes examining a dental defect in an oral cavity, performing a minimally invasive procedure to detect or remove at least a portion of the dental defect, scanning, responsive to the performing, the oral cavity including a residual tooth substance remaining after the performing, to obtain an intraoral scan, computing one or more constraints for one or more restoration materials, generating a plurality of treatment plans for the one or more restoration materials based on triangulation of the dental defect and enforcement of the one or more constraints of the one or more restoration materials, each treatment plan including a geometry of a restoration option and selecting from the plurality of treatment plans an optimal restoration option. The method may also include, for the minimally invasive procedure, an excavation of tooth material or a detection of dental caries.

In one aspect, a system is disclosed that includes a processor. The system also includes a memory storing instructions that, when executed by the processor, configure the apparatus to examine a dental defect in an oral cavity, perform a minimally invasive procedure to detect or remove at least a portion of the dental defect, scan, responsive to the performing, the oral cavity including a residual tooth substance remaining after the performing, to obtain an intraoral scan, compute one or more constraints for one or more restoration materials, generate a plurality of treatment plans for the one or more restoration materials based on triangulation of the dental defect and enforcement of the one or more constraints of the one or more restoration materials, each treatment plan including a geometry of a restoration option, and select from the plurality of treatment plans an optimal restoration option.

In yet another aspect, a non-transitory computer-readable storage medium is including instructions that when executed by a computer, cause the computer to perform the methods described herein. Even further, any of the methods described herein may be performed automatically with the aid of one or more processors.

Other technical features may be readily apparent to one skilled in the art from the following figures, descriptions, and claims.

### BRIEF DESCRIPTION OF THE DRAWINGS

To easily identify the discussion of any particular element or act, the most significant digit or digits in a reference number refer to the figure number in which that element is first introduced.
FIG. 1 depicts a block diagram of a network of data processing systems in accordance with an illustrative embodiment.
FIG. 2 depicts a block diagram of a data processing system in accordance with an illustrative embodiment.
FIG. 3 depicts a cross section of a restoration of a tooth illustrating wall thickness constraints in accordance with an illustrative embodiment.
FIG. 4 depicts a flowchart of a routine in accordance with an illustrative embodiment.
FIG. 5A depicts cross section of a minimally invasively prepared tooth in accordance with an illustrative embodiment.
FIG. 5B depicts cross section of a minimally invasively prepared tooth in accordance with an illustrative embodiment.
FIG. 5C depicts cross section of a minimally invasively prepared tooth in accordance with an illustrative embodiment.
FIG. 5D depicts cross section of a minimally invasively prepared tooth in accordance with an illustrative embodiment.
FIG. 5E depicts cross section of a minimally invasively prepared tooth in accordance with an illustrative embodiment.
FIG. 6 depicts a block diagram of an example configuration for intelligent proposal of restorations and in accordance with an illustrative embodiment.
FIG. 7 depicts a block diagram of an example training architecture for machine-learning based recommendation engine with an illustrative embodiment.
FIG. 8 depicts restoration preferences in accordance with an illustrative embodiment.

### DETAILED DESCRIPTION

The illustrative embodiments recognize that dental treatment planning may depend on limitations such as variability in practitioner expertise, subjectivity in diagnosis, and timeconsuming processes. Furthermore, integration of diverse diagnostic data sources, patient records, and treatment options may be challenging without a judicious approach that takes into consideration all possible options for treatment, constraints related to implementing the options, patient specific data and input preferences.

The illustrative embodiments disclose examining a dental defect in an oral cavity, performing a minimally invasive procedure to detect or remove at least a portion of the dental defect, and scanning, responsive to the performing, the oral cavity including a residual tooth substance remaining after the performing, to obtain an intraoral scan. The illustrative embodiments compute one or more parameters of the dental defect or residual tooth substance, generating a plurality of treatment plans for one or more restoration materials based on triangulation of the dental defect and enforcement of constraints of the one or more restoration materials, each treatment plan including a geometry of a restoration option and selecting from the plurality of treatment plans an optimal restoration option.

The illustrative embodiments are described with respect to certain types of machines. The illustrative embodiments are also described with respect to other scenes, subjects, measurements, devices, data processing systems, environments, components, and applications only as examples. Any specific manifestations of these and other similar artifacts are not intended to be limiting to the disclosure. Any suitable manifestation of these and other similar artifacts can be selected within the scope of the illustrative embodiments.

Furthermore, the illustrative embodiments may be implemented with respect to any type of data, data source, or access to a data source over a data network. Any type of data storage device may provide the data to an embodiment of the disclosure, either locally at a data processing system or over a data network, within the scope of the disclosure. Where an embodiment is described using a mobile device, any type of data storage device suitable for use with the mobile device may provide the data to such embodiment, either locally at the mobile device or over a data network, within the scope of the illustrative embodiments.

The illustrative embodiments are described using specific code, hardware, algorithms, designs, architectures, protocols, layouts, schematics, and tools only as examples and are not limiting. Furthermore, the illustrative embodiments are described in some instances using particular software, tools, and data processing environments only as an example for the clarity of the description. The illustrative embodiments may be used in conjunction with other comparable or similarly purposed structures, systems, applications, or architectures. For example, other comparable devices, structures, systems, applications, or architectures therefor, may be used in conjunction with such embodiment of the disclosure within the scope of the disclosure. An illustrative embodiment may be implemented in hardware, software, or a combination thereof.

The examples in this disclosure are used only for the clarity of the description and are not limiting to the illustrative embodiments. Additional data, operations, actions, tasks, activities, and manipulations will be conceivable from this disclosure and the same are contemplated within the scope of the illustrative embodiments.

Any advantages listed herein are only examples and are not intended to be limiting to the illustrative embodiments. Additional or different advantages may be realized by specific illustrative embodiments. Furthermore, a particular illustrative embodiment may have some, all, or none of the advantages listed above.

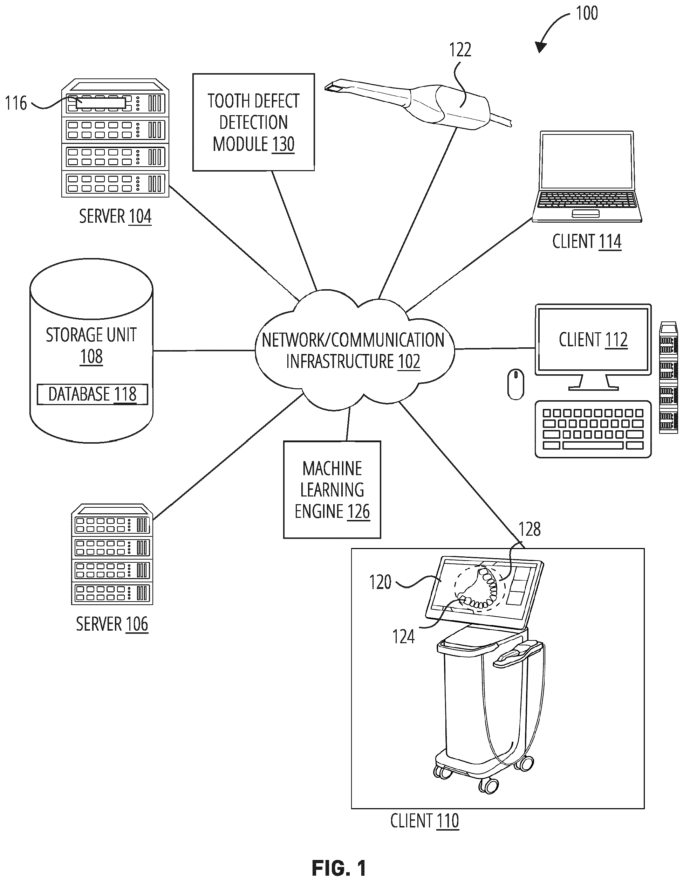

FIG. 1 depicts a block diagram of an environment of data processing systems in which illustrative embodiments may be implemented. Data processing environment 100 is a network of computers in which the illustrative embodiments may be implemented. Data processing environment 100 includes network/communication infrastructure 102. Network/communication infrastructure 102 is the medium used to provide communications links between various devices, databases and computers connected together within data processing environment 100. Network/communication infrastructure 102 may include connections, such as wire, wireless communication links, or fiber optic cables.

Clients or servers are only example roles of certain data processing systems connected to network/communication infrastructure 102 and are not intended to exclude other configurations or roles for these data processing systems. Server 104 and server 106 couple to network/communication infrastructure 102 along with storage unit 108. Software applications may execute on any computer in data processing environment 100. Client 110, client 112, client 114 are also coupled to network/communication infrastructure 102. Client 110 may be a dental acquisition unit with a display. A dental acquisition unit may be a device that generates and displays images of the inside of the oral cavity to create 3D models for dental restorations. A data processing system, such as server 104 or server 106, or clients (client 110, client 112, client 114) may include data and may have software applications or software tools executing thereon. It will be appreciated that the functionality of the server 104 or the server 106 could be implemented on one or more physical servers. Furthermore, functionality of the servers 104 and 106 could be implemented by a single server or group of servers operating together.

Only as an example, and without implying any limitation to such architecture, FIG. 1 depicts certain components that are usable in an example implementation of an embodiment. For example, servers and clients are only examples and do not imply a limitation to a client-server architecture. As another example, an embodiment can be distributed across several data processing systems and a data network as shown, whereas another embodiment can be implemented on a single data processing system within the scope of the illustrative embodiments. Data processing systems (server 104, server 106, client 110, client 112, client 114) also represent example nodes in a cluster, partitions, and other configurations suitable for implementing an embodiment.

Intra-oral camera 122 includes one or more sensors, such as separate sensors, which capture surfaces of tooth and defects in tooth.

Client application 120 or server application 116 (also referred to herein as a program) implement an embodiment described herein. Client application 120 and/or server application 116 can use data from intra-oral camera 122 for generating data related to tooth defect geometry (e.g. via tooth defect triangulation) and generate restoration options. Client application 120 can also execute in any of data processing systems (server 104 or server 106, client 110, client 112, client 114), such as client server application 116 in server 104 and need not execute in the same system as client 110.

Machine learning engine 126 may compute an optimal restoration option for tooth defects by classifying characteristics of a generated plurality of possible treatment plans. The machine learning engine 126 may store the treatment plans, or the treatment plans may be stored on a separate data store. Machine learning engine 126 may be a part of, or separate from server 104 or clients 110, 112 and 114. The machine learning engine 126 may be trained based on characteristics of a plurality of test treatment plans. As an example, the machine learning engine 126 may use a neural network. In such an example, the treatment plans (e.g. an attribute set including restoration type, material class, manufacturing process like printing or milling, manufacturing detail level) are the outputs of the output layer of neural network; whilst defect geometry and the other parameters like e.g. preferences (costs, time, durability, invasiveness) are the inputs fed into the input layer of the neural network. In such an example, only the perceptron weights would be stored for a trained network. As an example, training could be done as supervised training, e.g. initially based on dedicated dental university studies and, during daily use, using the feedback of the user (mainly if any other treatment than proposed is chosen). Training data bundles (input/output/human evaluation) can be stored to any type of database, no matter whether e.g. relational or object-oriented.

Server 104, server 106, storage unit 108, client 110, client 112, client 114, may couple to network/communication infrastructure 102 using wired connections, wireless communication protocols, or other suitable data connectivity. Client 110, client 112 and client 114 may be, for example, personal computers or network computers.

In the depicted example, server 104 may provide data, such as boot files, operating system images, and applications to client 110, client 112, and client 114. Client 110, client 112 and client 114 may be clients to server 104 in this example. Client 110, client 112 and client 114 or some combination thereof, may include their own data, boot files, operating system images, and applications. Data processing environment 100 may include additional servers, clients, and other devices that are not shown. Server 104 includes the server application 116 that may be configured to implement one or more of the functions described herein for displaying restoration proposals in accordance with one or more embodiments.

Server 106 may include a search engine configured to search stored files such as images of patient teeth for comparison in response to a request for detecting tooth defects. In another advantageous embodiment, the server contains a database of images including a specific identifier in the form of a vector representation of each image. In the depicted example, data processing environment 100 may be the Internet. Network/communication infrastructure 102 may represent a collection of networks and gateways that use the Transmission Control Protocol/Internet Protocol (TCP/IP) and other protocols to communicate with one another. At the heart of the Internet is a backbone of data communication links between major nodes or host computers, including thousands of dental practices, commercial, governmental, educational, and other computer systems that route data and messages (e.g. anonymized /pseudonymized data). Of course, data processing environment 100 also may be implemented as a number of different types of networks, such as for example, an intranet, a local area network (LAN), or a wide area network (WAN). FIG. 1 is intended as an example, and not as an architectural limitation for the different illustrative embodiments.

Among other uses, data processing environment 100 may be used for implementing a client-server environment in which the illustrative embodiments may be implemented. A client-server environment enables software applications and data to be distributed across a network such that an application functions by using the interactivity between a client data processing system and a server data processing system. Data processing environment 100 may also employ a service-oriented architecture where interoperable software components distributed across a network may be packaged together as coherent business applications. Data processing environment 100 may also take the form of a cloud, and employ a cloud computing model of service delivery for enabling convenient, on-demand network access to a shared pool of configurable computing resources (e.g. networks, network bandwidth, servers, processing, memory, storage, applications, virtual machines, and services) that can be rapidly provisioned and released with minimal management effort or interaction with a provider of the service.

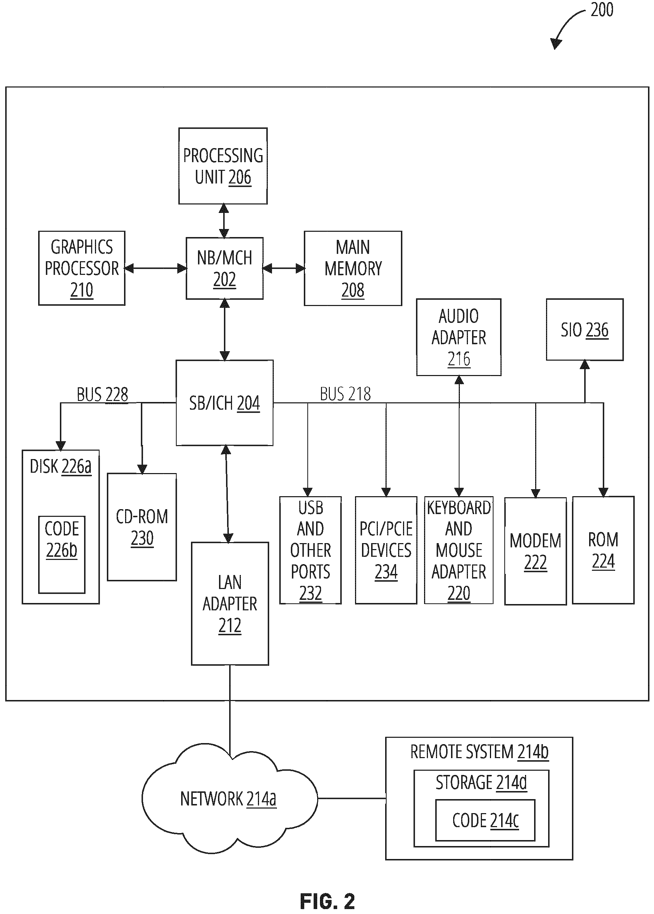

With reference to FIG. 2, this figure depicts a block diagram of a data processing system in which illustrative embodiments may be implemented. Data processing system 200 is an example of a computer, such client 110, client 112, client 114 or server 104, server 106, in FIG. 1, or another type of device in which computer usable program code or instructions implementing the processes may be located for the illustrative embodiments.

Data processing system 200 is described as a computer only as an example, without being limited thereto. Implementations in the form of other devices, may modify data processing system 200, such as by adding a touch interface, and even eliminate certain depicted components from data processing system 200 without departing from the general description of the operations and functions of data processing system 200 described herein.

In the depicted example, data processing system 200 employs a hub architecture including NorthBridge and memory controller hub (NB/MCH) 202 and SouthBridge and input/output (I/O) controller hub (SB/ICH) 204. Processing unit 206, main memory 208, and graphics processor 210 are coupled to North Bridge and memory controller hub (NB/MCH) 202. Processing unit 206 may include one or more processors and may be implemented using one or more heterogeneous processor systems. Processing unit 206 may be a multi-core processor. Graphics processor 210 may be coupled to North Bridge and memory controller hub (NB/MCH) 202 through an accelerated graphics port (AGP) in certain implementations.

In the depicted example, local area network (LAN) adapter 212 is coupled to South Bridge and input/output (I/O) controller hub (SB/ICH) 204. Audio adapter 216, keyboard and mouse adapter 220, modem 222, read only memory (ROM) 224, universal serial bus (USB) and other ports 232, and PCI/PCIe devices 234 are coupled to South Bridge and input/output (I/O) controller hub (SB/ICH) 204 through bus 218. Hard disk drive (HDD) or solid-state drive (SSD) 226a and CD-ROM 230 are coupled to South Bridge and input/output (I/O) controller hub (SB/ICH) 204 through bus 228. PCI/PCIe devices 234 may include, for example, Ethernet adapters, add-in cards, and PC cards for notebook computers. PCI uses a card bus controller, while PCIe does not. Read only memory (ROM) 224 may be, for example, a flash binary input/output system (BIOS). Hard disk drive (HDD) or solid-state drive (SSD) 226a and CD-ROM 230 may use, for example, an integrated drive electronics (IDE), serial advanced technology attachment (SATA) interface, or variants such as external-SATA (eSATA) and micro- SATA (mSATA). A super I/O (SIO) device 236 may be coupled to South Bridge and input/output (I/O) controller hub (SB/ICH) 204 through bus 218.

Memories, such as main memory 208, read only memory (ROM) 224, or flash memory (not shown), are some examples of computer usable storage devices. Hard disk drive (HDD) or solid-state drive (SSD) 226a, CD-ROM 230, and other similarly usable devices are some examples of computer usable storage devices including a computer usable storage medium.

An operating system runs on processing unit 206. The operating system coordinates and provides control of various components within data processing system 200 in FIG. 2. The operating system may be a commercially available operating system for any type of computing platform, including but not limited to server systems, personal computers, and mobile devices. An object oriented or other type of programming system may operate in conjunction with the operating system and provide calls to the operating system from programs or applications executing on data processing system 200.

Instructions for the operating system, the object-oriented programming system, and applications or programs, such as server application 116 and client application 120 in FIG. 1, are located on storage devices, such as in the form of codes 226b on Hard disk drive (HDD) or solid-state drive (SSD) 226a, and may be loaded into at least one of one or more memories, such as main memory 208, for execution by processing unit 206. The processes of the illustrative embodiments may be performed by processing unit 206 using computer implemented instructions, which may be located in a memory, such as, for example, main memory 208, read only memory (ROM) 224, or in one or more peripheral devices.

Furthermore, in one case, code 226b may be downloaded over network 214a (such as network/communication infrastructure 102) from remote system 214b, where similar code 214c is stored on a storage device 214d in another case, code 226b may be downloaded over network 214a to remote system 214b, where downloaded code 214c is stored on a storage device 214d.

A communications unit may include one or more devices used to transmit and receive data, such as a modem or a network adapter. A memory may be, for example, main memory 208 or a cache, such as the cache found in North Bridge and memory controller hub (NB/MCH) 202. A processing unit may include one or more processors or CPUs.

Where a computer or data processing system is described as a virtual machine, a virtual device, or a virtual component, the virtual machine, virtual device, or the virtual component operates in the manner of data processing system 200 using virtualized manifestation of some or all components depicted in data processing system 200. For example, in a virtual machine, virtual device, or virtual component, processing unit 206 is manifested as a virtualized instance of all or some number of hardware processing units 206 available in a host data processing system, main memory 208 is manifested as a virtualized instance of all or some portion of main memory 208 that may be available in the host data processing system, and Hard disk drive (HDD) or solid-state drive (SSD) 226a is manifested as a virtualized instance of all or some portion of Hard disk drive (HDD) or solid-state drive (SSD) 226a that may be available in the host data processing system. The host data processing system in such cases is represented by data processing system 200.

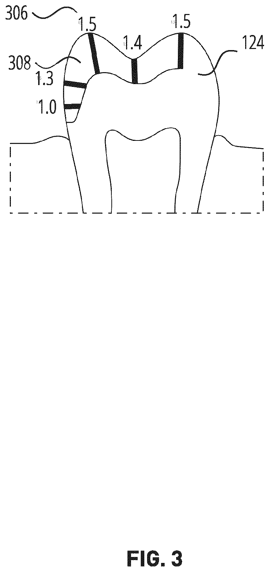

FIG. 3 illustrates a cross-sectional view of a tooth 124 with a restoration 308 (in this case, an onlay). The illustrative embodiments recognize that prior to placement of the onlay, the tooth 124 may be prepared to excavate any defects such as caries or holes in the tooth material. Depending on the material to be used for the restoration 308, manufacturer instructions may be followed to enforce constraints on the material including, for example, a minimum wall thickness 306 at different sections of the restoration 308. The remaining tooth material is thus shaped to be able to accommodate the constraints of the restoration material before installing the restoration 308.

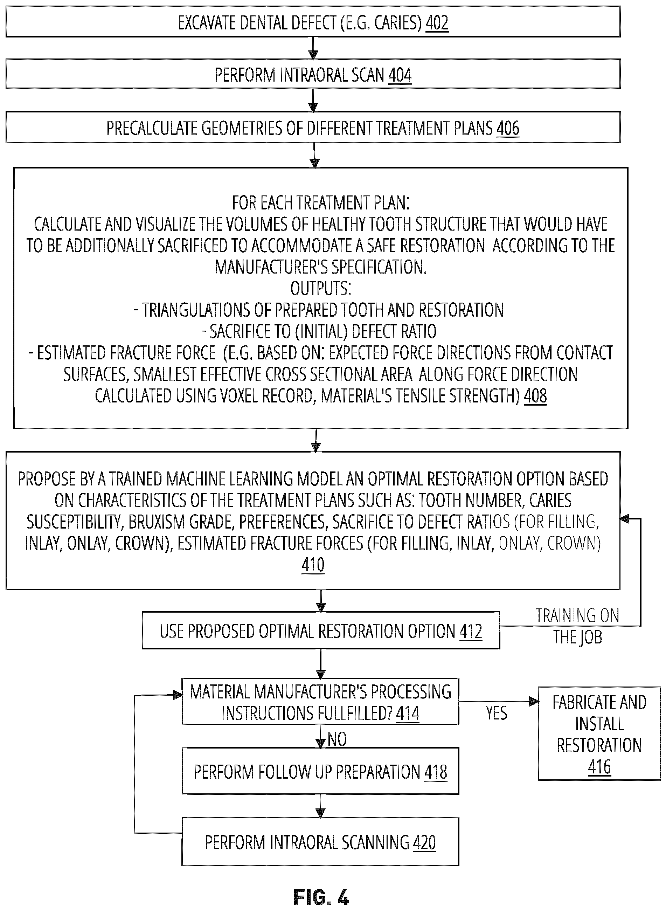

Turning now to FIG. 4, a method for generating an optimal restoration option is shown. The method includes performing a minimally invasive procedure at block 402, such as excavating, a dental defect of a patient's oral cavity. The defect may comprise, for example, caries, a hole in a tooth, or a broken tooth. The tooth defect may also comprise a tooth with a purposely removed part. The purposely removed part can be parts of tooth which has been excavated to remove diseased dental tissue. The method further includes scanning, at block 404, , the oral cavity including a residual tooth substance remaining, to obtain an intraoral scan.

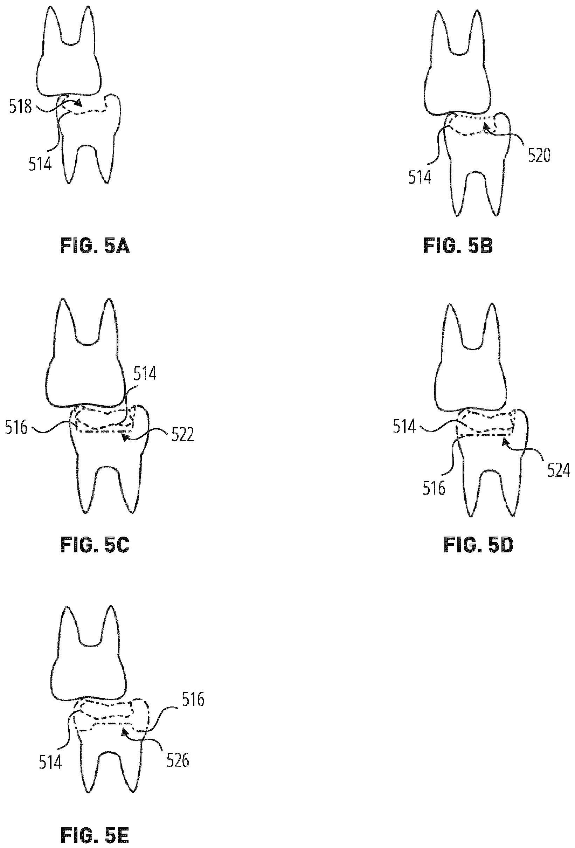

Treatment plans may be generated by computing one or more constraints for one or more restoration materials that can be used to produce restorations 308 to correct the defects. Constraints may also include constraints of the individual patient such as tooth/defect position. The treatment plans may further be generated by performing a triangulation of the dental defects (such as a volume occupied by the dental defect 518, see FIG. 5A) and enforcing the one or more constraints of the one or more restoration or restoration materials to be used in restoring the tooth. Each treatment plan may thus include a geometry of a restoration option. An optimal restoration option may then be selected generated treatment plans according to one or more aspects described herein.

Turning back to FIG. 4, in block 406, geometries of a number of treatment plans may be computed to illustrate to a patient or a dentist options for creating and installing a restoration, as shown in FIG. 5A - FIG. 5E, and specifically in FIG. 5B - FIG. 5E which illustrate restoration options of a plastic filling 520 (FIG. 5B), an inlay 522 (FIG. 5C), an onlay 524 (FIG. 5D), and a crown 526 (FIG. 5E). In the visualization of the restoration options of FIG. 5B - FIG. 5E, a defect boundary 514 is shown illustrating the edges of the defect. Further, a restoration boundary 516 is shown in FIG. 5C to FIG. 5E illustrating the edges of different types of restorations that would be implemented to conform to the constraints used. In FIG. 5B, the edges of the restoration may be the same as the edges of the volume occupied by the dental defect 518 after removal of the dental defect. Constraints considered in generating the geometries of the restoration options may include, for example, manufacturer instructions regarding restoration materials such as a minimum wall thicknesses of the one or more restoration materials, as well as constraints of the restoration such as additional volume of the residual tooth substance to remove after initial preparation of the tooth that had the defect to accommodate the restoration geometry, an expected masticatory pressure based on tooth position or defect location, and a medical history of the patient.

As shown in block 408 of FIG. 4, for every restoration option, the volumes of additional healthy tooth structure that would have to be sacrificed to accommodate a safe restoration according to the constraints may be computed. This may involve the computation of the triangulations/surface mesh of prepared tooth and restoration, for visualization. This may also involve the computation of a sacrifice to initial defect ratio, illustrating a ratio of the volume of the dental defect to the volume of tooth material being sacrificed for the restoration. An estimated fracture force for the restoration may also be computed. This may be based on, for example, expected force directions determined by contacting tooth surfaces, smallest effective cross section area along a force direction computed using, for example, voxel records, and a tensile strength of the restoration material.

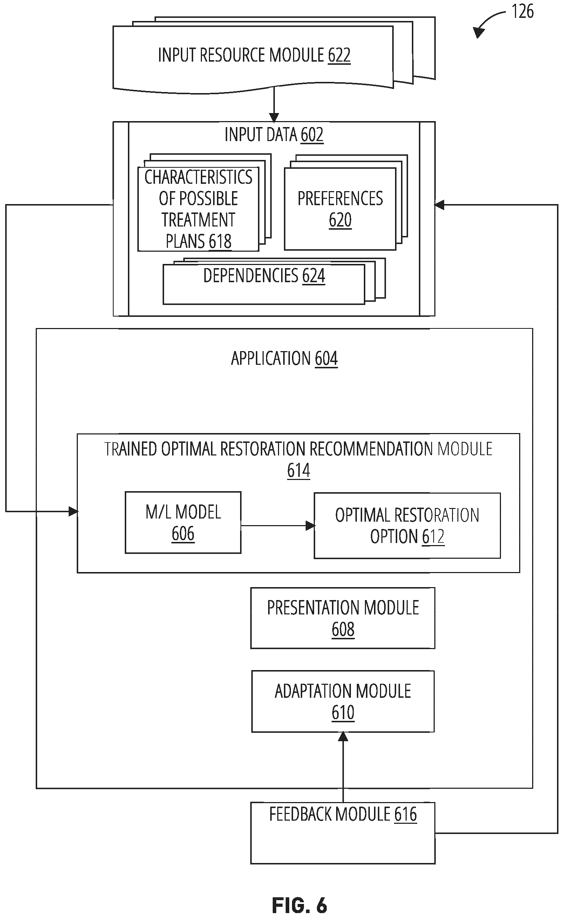

The computed parameters may then be used for the visualization and/or as part of one or more inputs to a proposal module used in selecting an optimal restoration option from the possible restoration options visualized, as shown in block 410. The proposal module may be based on, for example, statistical computations, artificial intelligence, model simulations and/or patient preferences. FIG. 6 - FIG. 7 illustrate an example wherein the proposal module is a machine learning engine 126. Herein, the machine learning engine 126 may comprise a trained machine learning model configured to propose an optimal restoration option based on characteristics of the plurality of treatment plans such as: tooth number, caries susceptibility, bruxism grade, patient preferences, sacrifice to defect ratios (for the filling, inlay, onlay, crown and any other restoration options visualized), estimated fracture forces (for the filling, inlay, onlay, crown and any other restoration options visualized). The trained machine learning model may then output information about each of the restoration options for use in determining the optimal restoration option or may highlight the optimal restoration option for the user based on such an output. The output parameters may include a durability of each restoration option such as a number of years each restoration option is expected to last.

As an example, the AI-implemented system may obtain intraoral scan data based on one or more intraoral scans of the dental cavity, and may identify at least one dental defect. The intraoral scan(s) may represent one or more images. Hence, the intraoral scan being of an oral cavity of a patient that includes a dental defect.

The intraoral scan data may then be encoded by determining a numerical representation such as a vector representation of an image of the dental defect. The numerical/vector representation may be fed to an input layer of neural network in the machine learning model.

Then, in the processing layers of the neural network, the method may comprise comparing/classifying the identifier/numerical representation of the dental defect to the identifiers/numerical identifiers in the database.

Then, in an output layer, the method may comprise determining at least one treatment option based on the highest similarity of the numerical representation of the scanned image to the numerical representation of the images in the database. Hence, the method may comprise selecting from the plurality of treatment plans at least one an optimal restoration option. In an embodiment, the numerical representation of the scanned image may be inputted into the input layer of the AI model and compared to prestored reference vector (or numerical) representations of dental defects to determine a recommendation, such as a score of a single treatment plan.

In another advantageous embodiment, the multiple different numerical representations of various treatment plans (e.g. a plastic crown model and a ceramic inlay model) may be inputted into the input layer of the AI model together with a reference vector representations or numerical representations of dental defects to determine a recommendation of several treatment plans which may be identified in the model and outputted by the output layer. A recommendation rank or a total recommendation can be determined in the output layer, and linked to each outputted possible treatment plan from the output layer. The recommendation score may provide an independent recommendation/suitability of each treatment plan. Optionally, a relative rating among the outputted possible treatment plans may be determined.

This method can be performed based on an initial scan of the dental effect, and or based on a scan performed after the diseased dental tissue has been removed (e.g. by a dentist). The model is trained on a large dataset of scanned images paired with at least one treatment option.

In some embodiments, the generating the plurality of treatment plans comprises determining a vector representation of the dental defect, and comparing the vector representation of the dental defect to prestored reference vector representations of dental defects.

As discussed, embodiments can obtain intraoral scan data from an intraoral scan, the intraoral scan being of an oral cavity of a patient that includes a dental defect; compute one or more constraints for one or more restoration materials for the dental defect; use the intraoral scan data to generate a plurality of treatment plans for the one or more restoration materials based on geometry of the dental defect and enforcement of the one or more constraints of the one or more restoration materials, each treatment plan including a geometry of a restoration option; and select from the plurality of treatment plans an optimal restoration option.

As mentioned above, in some embodiments, the geometry (or morphology) of the dental defect is determined by a triangulation or surface tessellation of the dental defect.

As an example, in the context of using a convolutional neural network, as a first step, the triangulation or tessellation may be transformed to a fixed-size representation in order to fit into a fixed size portion of the network's input layer, by means of e.g.:
- a histogram of vertex coordinates
- a bag-of-features representation (feature vector)
- or just remodeled representation with a fixed size of vertexes with their spatial components (x, y, z)

Hence, a next step may be to transform the triangulation or tessellation to a fixed-size representation in order to fit into a fixed size portion of the network's input layer, by means of e.g.:
- a histogram of vertex coordinates
- a bag-of-features representation (feature vector)
- or just remodeled representation with a fixed size of vertexes with their spatial components (x, y, z)

Then this data may be fed, together with other input variables (such as bruxism grade, tooth number, repair material type, process type like milling or printing, and all the others described above) into to the input layer.

In some embodiments, at least two triangulations or tessellations may be used. Such embodiments may calculate triangulation or tessellation of the digitized minimal-invasively prepared tooth, and then calculate a second triangulation or tessellation of the result of the treatment plan (i.e. the resulting replacement part body). In such cases, both triangulations or tessellations would be ideally located in the very same global coordinate system.

Then, the neural network may estimate the treatment plans' individual output parameters like invasiveness, costs, as well a total "recommendation level" (e.g. a number between 0 and 1). This step may then be repeated for each and every possible treatment plan. The "recommendation level" may relate to a combination of restoration type and restoration material type (e.g. plastic, ceramics, etc.).

The individual surface tessellation and location (relative to the tooth to be repaired) may be precalculated in advance by means of common volumetric CAD-algorithms, e.g. dilation of the original defect's volume in order to fulfil minimum wall thickness constraints and so on and so forth. The result may be a set of treatment options, each with a grade of recommendation, which can put them into relation by ranking them using the grade of recommendation.

As shown in FIG. 4, responsive to an operator agreeing to use a proposed optimal restoration option or selecting their own restoration as shown in block 410, the selection may be used as feedback to further train the machine learning model.

The operator may then perform an analysis of the intraoral cavity, in block 414, to determine if the constraints of the restoration/restoration material of the optimal restoration option will be met upon installation. The analysis may be performed by, for example, automatic computation based on comparison of volume information from the intra oral scan of block 404 with volume information about the optimal restoration option selected. Upon determining that further tooth material may have to be removed to meet the constraints, the operator may perform a follow up preparation in block 418 and obtain new intraoral scan in block 420. Responsive to determining that the constraints will be fulfilled, the optimal restoration option may be fabricated and installed in the oral cavity (block 416).

In some embodiments, the computation may involve a 2-step model with a first step to analyse the initial defect and then a second step to analyse the treatment options after the dentist has removed the tooth material.

In addition, some embodiments may involve performing a scan before preparing anything and then performing a second scan after a dental excavation (e.g. a minimal excavation). Then, after a suitable a morphologic operation, a full representation of the original geometric defect could be determined.

With reference to FIG. 6, this figure depicts a block diagram of an example configuration for intelligent proposal of an optimal restoration option in accordance with an illustrative embodiment. Application 604 is an example of any of server applications 116 or client application 120 in FIG. 1, depending on the particular implementation.

In one aspect, application 604 may receive characteristics of a plurality of possible treatment plans as input data 602. In another aspect, the machine learning engine 126 may receive the plurality of treatment plans as three-dimensional (3D) images, and identify, by an input resource module 622, the characteristics of the plurality of treatment plans for use as input data to the application 604. Even further, the input resource module 622 may receive the intraoral scan, compute from the intraoral scan the plurality of treatment plans, and compute therefrom characteristics of the plurality of treatment plans. As discussed herein, the characteristics of possible treatment plans 618 may include a tooth number of the defective tooth, a caries susceptibility of the patient, a bruxism grade of the patient (such as one rated on a relative scale of normal, increased, heavy) preferences 620 ( such as costs, conservation, durability preference), computed sacrifice to defect ratios (of the plurality of treatment plans including, for example, the plastic filling 520, inlay 522, onlay 524, crown 526), estimated fracture forces (of the plurality of treatment plans including, for example, the plastic filling 520, inlay 522, onlay 524, crown 526). These may further be computed for different material types for each restoration option.

The input data may be used as input for a trained optimal restoration recommendation module 614 and the trained optimal restoration recommendation module 614 may extract, responsive to the identifying, one or more features from the input data, the one or more features representative of a request for completing the recommendation process. The trained optimal restoration recommendation module 614 may then propose at least one optimal restoration option 612 needed to achieve a desired restoration installation taking into consideration specific patient parameters that may be present for one patient and that may be absent for another patient.

The input resource module 622 may be a dental software component that retrieves and/or prepares the characteristics of possible treatment plans 618, from any source such as the intra-oral camera 122 from and/or other sources. The input data 602 may be representative of each of the possible treatment plans and may further include preferences 620 obtained from, for example, a patient profile, a practitioner profile, a group profile, etc. Dependencies 624 such as availability of device types, connected devices, etc. may also be used as input data 602.

Further, individual portions of the input data 602 may be weighted or prioritized to drive corresponding changes in proposals. These dependencies may otherwise be complex, need significant skill when chosen manually and their significance on final user-specific restoration options may not always be clearly understood by every operator. Thus, not taking said dependencies and preferences into account may result in sub-optimal restoration results, processes, and cost.

In an embodiment, the trained optimal restoration recommendation module 614 (trained machine learning model) may be configured to include a feature extraction component that may generate relevant features for a proposal based on data from all the different available features (e.g., characteristics of possible treatment plans 618, preferences 620, dependencies 624). The feature extraction component may be part of the trained optimal restoration recommendation module 614, which may comprise be a deep neural network/ m/l model 606 (machine learning model). In other embodiments, the feature extraction component may be a feature selection component and may be separate from the trained optimal restoration recommendation module 614. The feature extraction component may use a defined algorithm of prioritization or dependencies to generate the features for the recommendation of the optimal restoration option 612.

As discussed herein, the trained optimal restoration recommendation module 614 may propose an optimal restoration option 612 which may include a durability of each of the optimal restoration options or a durability of all input restoration options with the optimal durability being highlighted.

The trained optimal restoration recommendation module 614 can be based, for example, on an artificial machine learning neural network such as a convolutional neural network (CNN), though it is not meant to be limiting. It may be a feed-forward artificial neural network which in a classic form may comprise a convolutional layer, followed by a pooling layer. The CNN learns by learning free parameters or classifiers of the convolution kernel per layer and their weighting when calculating the next layer.

A training of the m/l model 606 or trained optimal restoration recommendation module 614 according to an illustrative embodiment is discussed hereinafter.

In an illustrative embodiment, presentation module 608 of application 604 displays the output or optimal restoration option obtained from the trained optimal restoration recommendation module 614. The presentation module 608 may also display, for example, plurality of restoration options ranked from most optimal to least optimal for the specified user. An adaptation module 610 may be configured to receive input from an operator to adapt the optimal restoration option 612 if necessary. For example, changing a material preference may cause recalculation of proposed optimal restoration option 612 for presentation by the presentation module 608.

As new tools and materials with specific instructions and requirements are added to a dental workflow, and as operators continue to make decisions on optimal restoration options 612 proposed for them, the trained optimal restoration recommendation module 614 may be retrained and a need for the practitioner to be intimately aware of all user specific details may be significantly reduced. Feedback module 616 may further collect user feedback 620 indicative of an accuracy of the optimal restoration option 612.

The neural network m/l model 606 may be trained using various types of training data sets. The training may include using a training dataset that comprises training input characteristics of possible treatment plans, preferences and dependencies and corresponding training output optimal restoration options corresponding to the training input. The training input and output may be obtained or computed from a plurality of existing treatment databases or via expert analysis.

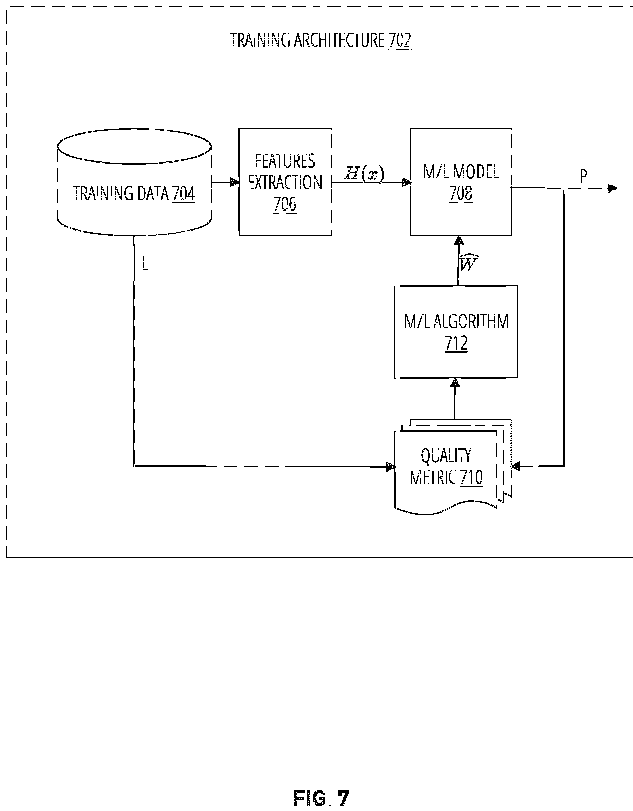

FIG. 7 shows an example training architecture 702 for machine-learning based proposal generation in accordance with an illustrative embodiment. Herein, program code extracts various features/attributes 706 from training data 704 with the training data entries having labels L. The features are utilized to develop a predictor function, H(x) or a hypothesis, which the program code utilizes as a machine learning model 708. In identifying various features/attributes in the training data 704, the program code may utilize various techniques including, but not limited to, mutual information, which is an example of a method that can be utilized to identify features in an embodiment. Other embodiments may utilize varying techniques to select features, including but not limited to, principal component analysis, diffusion mapping, a Random Forest, and/or recursive feature elimination (a brute force approach to selecting features), to select the features. "P" is the output (e.g., restoration materials parameters and/or probabilities) that can be obtained, which when received, could further trigger the dental system to perform other steps such as display options or start a machining process. The program code may utilize a machine learning algorithm 712 to train machine learning model 708, including providing weights for the outputs, so that the program code can prioritize various changes based on the predictor functions that comprise the machine learning model 708. The output can be evaluated by a quality metric 710.

By selecting a diverse set of training data 704, the program code trains machine learning model 708 to identify and weight various attributes of patients, practitioners, devices connected to the dental system, etc. To utilize the machine learning model 708, the program code obtains (or derives) input data or features to generate an array of values to input into input neurons of a neural network. Responsive to these inputs, the output neurons of the neural network produce an array that includes the output optimal restoration option that may be presented contemporaneously on a display.

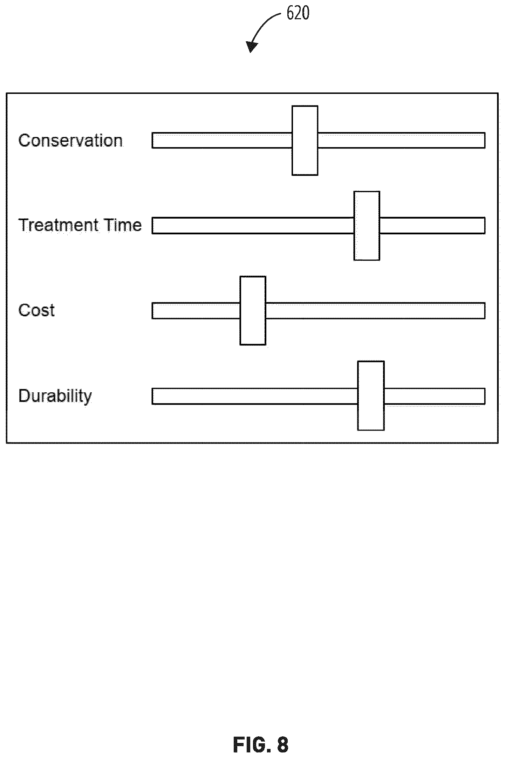

Turning now to FIG. 8, an example of preferences 620 is shown. An operator may thus select any number of values for conservation, treatment time, cost, and durability using, for example, a sliding scale.

In another aspect, a proximity of a residual tooth substance to a pulp or tooth root may be computed for use in computing an invasiveness score based on at least the proximity. This may further be used as input to the trained optimal restoration recommendation module 614. Further, as part of the output of the trained optimal restoration recommendation module 614, a restoration prognosis comprising a stability score and/or a predicted lifetime may be provided for each possible treatment plan.

In another aspect, at least one tooth number is used as one of the input data 602.

In another aspect, the proposal module may comprise analytical/numerical computation, statistical analysis, and preference filtering components.

Any specific manifestations of these and other similar example processes are not intended to be limiting to the disclosure. Any suitable manifestation of these and other similar example processes can be selected within the scope of the illustrative embodiments.

Thus, a computer-implemented method, system or apparatus, and computer program product are provided in the illustrative embodiments for generating optimal restoration options and other related features, functions, or operations. Where an embodiment or a portion thereof is described with respect to a type of device, the computer-implemented method, system or apparatus, the computer program product, or a portion thereof, are adapted or configured for use with a suitable and comparable manifestation of that type of device.

Where an embodiment is described as implemented in an application, the delivery of the application in a Software as a Service (SaaS) model is contemplated within the scope of the illustrative embodiments. In a SaaS model, the capability of the application implementing an embodiment is provided to a user by executing the application in a cloud infrastructure. The user can access the application using a variety of client devices through a thin client interface such as a web browser, or other light-weight client-applications. The user does not manage or control the underlying cloud infrastructure including the network, servers, operating systems, or the storage of the cloud infrastructure. In some cases, the user may not even manage or control the capabilities of the SaaS application. In some other cases, the SaaS implementation of the application may permit a possible exception of limited user-specific application configuration settings.

In the following, various examples for implementing aspects of the disclosure are described:
Example 1. A method comprising:
   examining a dental defect in an oral cavity;
   performing a minimally invasive procedure to detect or remove at least a portion of the dental defect;
   scanning, responsive to the performing, the oral cavity including a residual tooth substance remaining after the performing, to obtain an intraoral scan;
   computing one or more constraints for one or more restoration materials;
   generating a plurality of treatment plans for the one or more restoration materials based on triangulation of the dental defect and enforcement of the one or more constraints of the one or more restoration materials, each treatment plan including a geometry of a restoration option and
   selecting from the plurality of treatment plans an optimal restoration option.
Example 2. The method of example 1, or any other examples, wherein the minimally invasive procedure includes an excavation of tooth material or a detection of dental caries.
Example 3. The method of example 1, or any other examples, wherein the plurality of treatment plans are selected from the list consisting of a filling, an inlay, an onlay, and a crown.
Example 4. The method of example 3, or any other examples, further comprising:
   executing the optimal restoration option.
Example 5. The method of example 1, or any other examples, further comprising:
   proposing, by a trained machine learning model, said optimal restoration option using characteristics of the plurality of treatment plans as inputs to the trained machine learning model.
Example 6. The method of example 5, or any other examples, wherein the characteristics of the plurality of treatment plans are selected from the list consisting of a tooth number, a caries susceptibility, a bruxism grade, a patient or dentist preference, a sacrifice to defect ratio parameter for each treatment plan that represents a volume of tooth material to remove for the treatment plan relative to a volume of the dental defect, and an estimated fracture force for each treatment plan.
Example 7. The method of example 5, or any other examples, wherein the optimal restoration option includes a durability of a treatment plan of the plurality of treatment plans.
Example 8. The method of example 1, or any other examples, wherein the one or more constraints is selected from a list consisting of a minimum wall thickness of the one or more restoration materials, a volume of the residual tooth substance to remove to achieve a treatment plan, an expected masticatory pressure based on tooth position or defect location, and a medical history of a patient.
Example 9. The method of example 1, or any other examples, computing from the intraoral scan, responsive to selecting the optimal restoration option, an extent to which an area of the oral cavity to be restored meets manufacturer processing instructions for the material of the optimal restoration option, and
   responsive to determining that the extent is below a threshold, performing a follow-up preparation to further prepare the area according to the manufacturer processing instructions, fabricating the optimal restoration option, and installing the optimal restoration option.
Example 10. The method of example 1, or any other examples, further comprising:
   computing a volume of the residual tooth substance to remove to achieve a treatment plan and computing an invasiveness score based on at least the volume.
Example 11. The method of example 1, or any other examples, further comprising:
   for each treatment plan of the plurality of treatment plans displaying on a screen, a triangulation of a prepared tooth and/or a restoration of the treatment plan;
   displaying a sacrifice to defect ratio parameter representing a volume of material to remove relative to a volume of the dental defect; and
   displaying an expected fracture force of the restoration.
Example 12. The method of example 1, or any other examples, further comprising:
   computing a proximity of the residual tooth substance to a pulp or tooth root and computing an invasiveness score based on at least the proximity.
Example 13. The method of example 1, or any other examples, further comprising:
   for each treatment plan of the plurality of treatment plans, computing a restoration prognosis comprising a stability score and/or a predicted lifetime.
Example 14. The method of example 1, or any other examples, wherein the generating is further based on a preference selected from the list consisting of a material availability, a cost limit, a maximum invasiveness, a minimum restoration lifetime.
Example 15. The method of example 1, or any other examples, wherein the dental defect is visualized.
Example 16. The method of example 1, or any other examples, wherein the plurality of treatment plans are ordered according to a predefined metric and visualized.
Example 17. The method of example 16, or any other examples, wherein one or more other characteristics of the dental defect are also visualized.
Example 18. The method of example 16, or any other examples, wherein the generating is performed by one or more of machine learning, analytical/numerical computation, statistical analysis, and preference filtering.
Example 19. A system comprising:
   a processor; and
   a memory storing instructions that, when executed by the processor, configure the apparatus to:
      examine a dental defect in an oral cavity;
      perform a minimally invasive procedure to detect or remove at least a portion of the dental defect;
      scan, responsive to the performing, the oral cavity including a residual tooth substance remaining after the performing, to obtain an intraoral scan;
      compute one or more constraints for one or more restoration materials;
      generate a plurality of treatment plans for the one or more restoration materials based on triangulation of the dental defect and enforcement of the one or more constraints of the one or more restoration materials, each treatment plan including a geometry of a restoration option and
      select from the plurality of treatment plans an optimal restoration option.
Example 20. A non-transitory computer-readable storage medium, including instructions that when executed by a computer, cause the computer to:
   examine a dental defect in an oral cavity;
   perform a minimally invasive procedure to detect or remove at least a portion of the dental defect;
   scan, responsive to the performing, the oral cavity including a residual tooth substance remaining after the performing, to obtain an intraoral scan;
   compute one or more constraints for one or more restoration materials;
   generate a plurality of treatment plans for the one or more restoration materials based on triangulation of the dental defect and enforcement of the one or more constraints of the one or more restoration materials, each treatment plan including a geometry of a restoration option and select from the plurality of treatment plans an optimal restoration option.

The present disclosure may be a system, a method, and/or a computer program product at any possible technical detail level of integration. The computer program product may include a computer-readable storage medium (or media) having computer-readable program instructions thereon for causing a processor to carry out aspects of the present disclosure.

The computer-readable storage medium can be a tangible device that can retain and store instructions for use by an instruction execution device. The computer-readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer-readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer-readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer-readable program instructions described herein can be downloaded to respective computing/processing devices from a computer-readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer-readable program instructions from the network and forwards the computer-readable program instructions for storage in a computer-readable storage medium within the respective computing/processing device.

Computer-readable program instructions for carrying out operations of the present disclosure may be assembler instructions, instruction-set-architecture (ISA) instructions, machine instructions, machine dependent instructions, microcode, firmware instructions, statesetting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer-readable program instructions may execute entirely on a dedicated system or user's computer, partly on the user's computer or dedicated system as a stand-alone software package, partly on the user's computer and partly on a remote computer or entirely on the remote computer or server, etc. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer-readable program instructions by utilizing state information of the computer-readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer-readable program instructions.

These computer-readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer-readable program instructions may also be stored in a computer-readable storage medium that can direct a computer, a programmable data processing apparatus, and/or other devices to function in a particular manner, such that the computer-readable storage medium having instructions stored therein comprises an article of manufacture including instructions which implement aspects of the function/act specified in the flowchart and/or block diagram block or blocks.

The computer-readable program instructions may also be loaded onto a computer, other programmable data processing apparatus, or other device to cause a series of operational steps to be performed on the computer, other programmable apparatus or other device to produce a computer-implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

All features disclosed in the specification, including the claims, abstract, and drawings, and all the steps in any method or process disclosed, may be combined in any combination, except combinations where at least some of such features and/or steps are mutually exclusive. Each feature disclosed in the specification, including the claims, abstract, and drawings, can be replaced by alternative features serving the same, equivalent, or similar purpose, unless expressly stated otherwise.

## Claims

1. A system comprising:
a processor; and
a memory storing instructions that, when executed by the processor, configure the processor to:
obtain intraoral scan data from an intraoral scan, the intraoral scan being of an oral cavity of a patient that includes a dental defect;
compute one or more constraints for one or more restoration materials for the dental defect;
use the intraoral scan data to generate a plurality of treatment plans for the one or more restoration materials based on geometry of the dental defect and enforcement of the one or more constraints of the one or more restoration materials, each treatment plan including a geometry of a restoration option; and
select from the plurality of treatment plans an optimal restoration option.

2. The system of claim 1, wherein the dental defect comprises a tooth defect or a missing part of a tooth.

3. The system of claim 1 or 2, wherein the geometry of the dental defect is determined by triangulation or tessellation of the dental defect.

4. The system of any one of claims 1 to 3, wherein the plurality of treatment plans are selected from the list consisting of a filling, an inlay, an onlay, and a crown.

5. The system of any one of claims 1 to 4, wherein the generating the plurality of treatment plans comprises determining a vector representation of the dental defect, and comparing the vector representation of the dental defect to prestored reference vector representations of dental defects.

6. The system of claim 5, wherein the comparing the vector representation of the dental defect to prestored reference vector representations of dental defects comprises:
selecting a group of prestored reference vector representations of dental defects and comparing the vector representation of the dental defect to this group prestored reference vector representations of dental defects,
wherein the group of prestored reference vector representations is selected based on data related to the patient.

7. The system of any one of claims 1 to 6, wherein the processor is configured to:
propose, using a trained machine learning model, said optimal restoration option using characteristics of the plurality of treatment plans as inputs to the trained machine learning model;
optionally wherein the characteristics of the plurality of treatment plans are selected from the list consisting of a tooth number, a caries susceptibility, a bruxism grade, a patient or dentist preference, a sacrifice to defect ratio parameter for each treatment plan that represents a volume of tooth material to remove for the treatment plan relative to a volume of the dental defect, and an estimated fracture force for each treatment plan;
optionally wherein the optimal restoration option includes a durability of a treatment plan of the plurality of treatment plans.

8. The system of any one of claims 1 to 7, wherein the one or more constraints is selected from a list consisting of a minimum wall thickness of the one or more restoration materials, a volume of a residual tooth substance to remove to achieve a treatment plan, an expected masticatory pressure based on tooth position or defect location, and a medical history of a patient.

9. The system of any one of claims 1 to 8, wherein the processor is configured to:
compute a volume of the residual tooth substance to remove to achieve a treatment plan and computing an invasiveness score based on at least the volume.

10. The system of any one of claims 1 to 9, wherein the processor is configured to:
for each treatment plan of the plurality of treatment plans display on a screen, a triangulation or tessellation of a prepared tooth and/or a restoration of the treatment plan;
display a sacrifice to defect ratio parameter representing a volume of material to remove relative to a volume of the dental defect; and
display an expected fracture force of the restoration.

11. The system of any one of claims 1 to 10, wherein the processor is configured to:
use the intraoral scan data to compute a proximity of residual tooth substance to a pulp or tooth root and computing an invasiveness score based on at least the proximity.

12. The system of any one of claims 1 to 11, wherein the processor is configured to:
for each treatment plan of the plurality of treatment plans, compute a restoration prognosis comprising a stability score and/or a predicted lifetime; and/or
wherein the generating is further based on a preference selected from the list consisting of a material availability, a cost limit, a maximum invasiveness, a minimum restoration lifetime.

13. The method of any one of claims 1 to 12, wherein the plurality of treatment plans are ordered according to a predefined metric and visualized;
optionally wherein one or more other characteristics of the dental defect are also visualized;
optionally wherein the generating is performed by one or more of machine learning, analytical/numerical computation, statistical analysis, and preference filtering.

14. The system of any one of claims 1 to 13, further comprising a scanner arranged to perform the intraoral scan of the oral cavity.

15. A non-transitory computer-readable storage medium, including instructions that when executed by a computer, cause the computer to:
obtain intraoral scan data from an intraoral scan, the intraoral scan being of an oral cavity of a patient that includes a dental defect;
compute one or more constraints for one or more restoration materials for the dental defect;
use the intraoral scan data to generate a plurality of treatment plans for the one or more restoration materials based on geometry of the dental defect and enforcement of the one or more constraints of the one or more restoration materials, each treatment plan including a geometry of a restoration option; and
select from the plurality of treatment plans an optimal restoration option.
